# EUROPEAN PATENT SPECIFICATION

(11) **EP 0 483 212 B1**
(45) Date of publication and mention of the grant of the patent: **18.05.1994**
(21) Application number: 90910963.9
(22) Date of filing: 18.06.1990
(51) Int. Cl.: A61F 13/56

(54) **ABSORBENT DISPOSABLE ARTICLE**
Absorbierender Wegwerfartikel
ARTICLE ABSORBANT JETABLE

(30) Priority: 07.07.1989 SE 8902470
(43) Date of publication of application: 06.05.1992
(73) Proprietor: Mölnlycke AB, 405 03 Göteborg (SE)
(72) Inventor: WIDLUND, Urban, S-435 43 Mölnlycke (SE); HANSSON, Roy, S-435 45 Mölndal (SE)
(74) Representative: Kierkegaard, Lars-Olov
(86) International application number: SE9000429
(87) International publication number: WO9100721

(56) References cited:
- EP-A- 0 098 983
- EP-A- 0 215 408

## Description

The present invention relates to an absorbent disposable article, such as a diaper or an incontinence guard, comprising an inner permeable enveloping layer, which when the article is in use, lies closest to the user's body, an outer impermeable enveloping layer and an absorbent body attached between the enveloping layers.

Diapers and incontinence guards of so-called all-in-one type are nowadays usually fitted with leg and waist elastic in the form of elastic threads or elastic bands, which are attached to one or both of the two enveloping layers, which enclose the absorbent body in such products. This type of elastics increases the diaper's safety against leakage and gives them a better fit. However, this elastic which is placed on the enveloping layers around the absorbent body, can cause undesired deformation of the absorbent body, especially if this is thin, and also the threads in such elastic are attached in a prestretched state, which usually means that the elastic threads must be kept under tension during all the steps of the manufacturing process of such a diaper, which makes its manufacture complicated.

By EP-A-0 098 983, an all-in-one diaper is previously known, which consists of enveloping layers of elastic material enclosing an absorbent body. The intention of this construction is to simplify manufacture. A disadvantage of such a diaper is that it is difficult to constructively ensure adequate elastic tension around the legs and waist of the user, as the elastic tension only depends on the way the diaper is applied by whoever puts it on the user. There is therefore an obvious risk that such a diaper will be fitted too tight on the user.

A similar solution was suggested in US-A-4,699,621 ≙ EP-A-0,215,408, where a separate layer of elastic material according to this document is attached to the diaper in such a way that its elastic characteristics are essentially unchanged. Also in this type of diaper, the elastic tension is totally dependent on how the diaper is put on.

The present invention intends to solve the above mentioned problem without it being possible for the diaper or incontinence guard to be applied too tightly.

This object is accomplished according to the invention, by an absorbent disposable article, of the aforementioned kind characterized by a piece of a thin, flexible material being attached to the outer enveloping layer, the piece of material comprising a pattern of elastic threads or elastic bands, being attached to the piece of material in a pretensioned state, and this piece of material extending in the article's length-wise direction past the back edges of the enveloping layers of the absorbent body; and which in the article's breadth-wise direction in the back section has such an extension that when the article is placed on the user, the side edges of the piece of material are attachable to the corresponding side edge of the article's front section in order to give the applied article a pants-like configuration, whereby the pattern of elastic threads comprises threads which constitute leg and waist elastics.

By placing the elastic threads or elastic bands in a separate piece of material, as well as solving the above mentioned problem, several other advantages are obtained. Due to the elastic threads not being attached either to the absorbent body or to one of the enveloping layers attached to the absorbent body, their elastic characteristics can be utilized much better. Furthermore, the previously best known solution as regards leakage security for diapers, that is, diapers held tight to the diaper-child with the help of specially constructed elastic pants with waist and leg elastic, can be more closely emulated. The piece of material can be given any shape desired, and therefore it is not necessary for the sidepieces of the enveloping layers encasing the absorbent body to be attached to each other when the diaper is being used, as is the case with conventional all-in-one diapers. Moreover, the elastic threads being arranged in a separate piece of material gives a greater variation in size of the children for whom the diaper will give a good fit, compared with the conventional all-in-one diapers in which contraction of the elastic threads from the pretensioned, i.e. stretched, condition to the totally relaxed condition, is restricted by the absorbent body. Furthermore, the material in the material piece does not have to be impermeable, which means that a soft and pleasant material with good air-exchanging properties can be selected for the material piece.

A preferred embodiment of the invention will now be described in detail with reference to the accompanying drawings, in which:
Fig. 1 is a perspective view schematically showing a diaper according to the invention;
Fig. 2 is a plan view showing the diaper according to Fig. 1 with the elastic threads in a stretched state; and
Fig. 3 is a view similar to Fig. 2 showing a variation of the diaper.

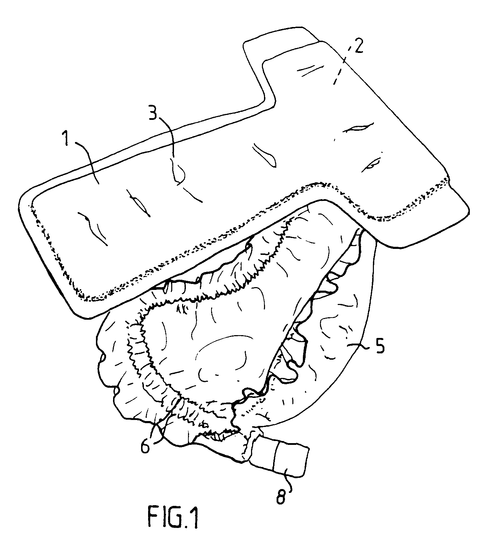

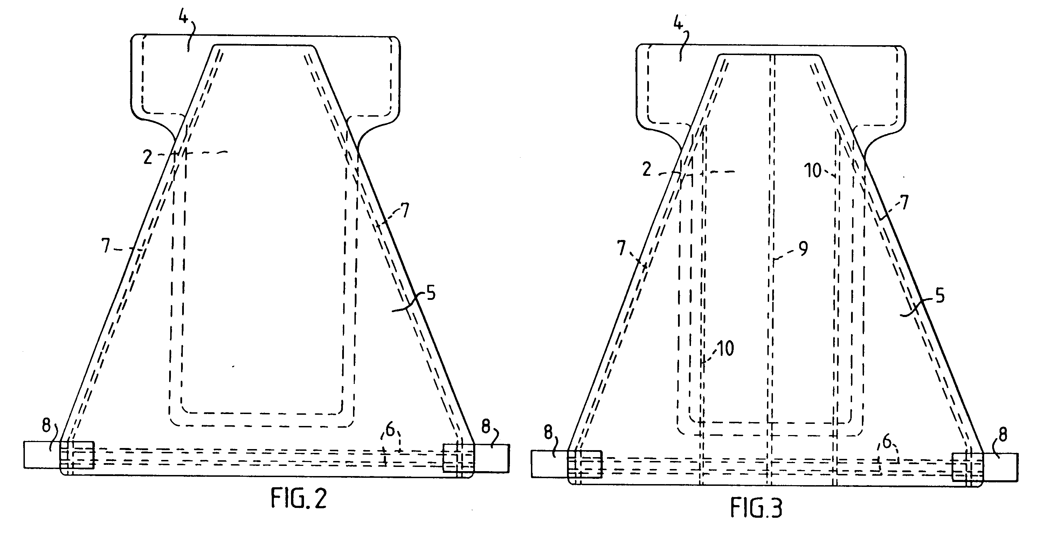

The diaper shown in the Figures comprises a conventional T-diaper with an absorbent body 2 encased between two enveloping layers 3, 4. The inner enveloping layer 3, which on usage of the diaper, lies against the user's body and which in Fig. 1 is turned towards the viewer, is constructed of a permeable material, preferably of a non-woven material such as non-woven fiber material. The outer enveloping layer 4, which in Fig. 2 is turned towards the viewer, is constructed of an impermeable material, for example polyethylene or polypropylene plastic. Both of these enveloping layers are joined in a suitable way, for example with an adhesive, at the parts extending beyond the absorbent body 2.

A material piece 5 is attached in a suitable way, for example by gluing, to the outer enveloping layer 4 of the T-diaper, in a narrow area close to the transverse edge of that area of the T-diaper, which contains the parts of the T-diaper forming the side sections and which on usage, form the diaper's front part, lying against the user's abdomen. The material piece 5 is symmetrically aligned relative to the T-diaper's 1 length-wise mid-line.

The material piece 5 has, as apparent from Fig. 2, in the shown version the form of a cut-off triangle and contains two transverse elastic threads 6, which are extended parallel to the edge of the material piece opposite the attachment edge. Furthermore, an elastic thread 7 is extended along each side edge of the material piece. Elastic threads 6 and 7 are in a suitable way attached to the material piece in a pretensioned condition. In the preferred version shown, the material piece 5 consists of two layers of non-woven material, between which threads 6 and 7 are arranged and attached at the same time as the layers were glued together. It is, of course, possible to form the material piece from a single material layer and attach the threads directly thereto. The threads 6,7 can also be attached in other ways than gluing, for example they can be sewn together.

The material piece 5 also comprises two tape-flaps 8 intended to join the side-parts of the material piece and the protruding side-parts of the T-diaper when the diaper is put on.

In Fig. 2 is shown the diaper according to the invention with elastic threads in the stretched, i.e. pretensioned, condition, while Fig. 1 schematically shows the diaper with the elastic threads contracted, i.e. in an unstretched or relaxed condition. As can be seen from Fig. 1, the contraction of the threads causes an extensive fold formation in the non-woven material. Due to the non-woven material's pliability permitting the elastic threads 6, 7 to be totally relaxed, the threads can return to their original length. On putting on a diaper according to the invention, the material piece 5 is thus pulled from its position as shown in Fig. 1, so that the fastening tapes 8 can be attached to the extended side-pieces of the T-diaper 1. When the inventive diaper is put on, as formed by T-diaper 1 and material piece 5, it assumes a pants-like configuration and the threads 6 and 7 respectively, form the waist and leg elastic bands. Compared to a conventional all-in-one diaper with elastic threads arranged between the enveloping layers encasing the absorbent body, the diaper according to the invention, affords greater adaptability to different sizes of diaper users, as the active length of the elastic threads in material piece 5 is much greater than the active length of the threads in a conventional all-in-one diaper because the absorbent body in such a diaper prevents constriction of the threads, especially in those threads which form the elastic at the waist. In a conventional all-in-one diaper, the active length of the waist elastic is determined to a considerable degree by the constriction of the elastic threads in the side-pieces of the enveloping layers, lying outside the absorbent body. It should be noted, in this connection, that even if the transverse elastic threads which form the waist elastic in an all-in-one diaper totally extend between the enveloping layers, which lie outside the absorbent body, this will still greatly restrict the threads' ability to contract. A diaper according to the invention therefore always permits better adjustment to different sizes of the diaper users than conventional all-in-one diapers on the condition that the material piece is dimensioned herefor, and that adequate values for the tension of the waist elastic are used. Furthermore, a diaper according to the invention has, as does the conventional all-in-one diaper, the advantage that maximum tensile strength can be easily determined by applying an adequate tension in the elastic threads.

Fig. 3 shows a version which differs from that shown in Fig. 2 by the presence of extra length-wise elastic threads 9, 10 attached to material piece 5, which when the diaper is put on, are extended within the area of the absorbent body in order to press this against the user's body. These threads should have a suitable, lower tension than the elastic threads 6, 7.

The embodiments as shown in the Figures can of course be modified in several ways within the limits of the invention. For example, the form of the material piece and the diaper to which it is attached, can be varied. If the invention is applied to a rectangular diaper it is advisable to use a material with extended side-pieces at that end which is attached to the outside of the rectangular diaper, and it can also then be advisable to provide both sides with transverse elastic which extends from side to side, to further increase the effective length of the waist elastic. The deformation of the absorbent body or of the outer enveloping layers of the diaper, which could occur due to the presence of the elastic in the attachment areas for the material piece, is small and has no effect on the functional characteristics of the diaper.

The extent of the invention should therefore only be restricted by the contents of the accompanying claims.

Further, it is of course possible to attach the material piece anywhere on the diaper on the condition that its back section can co-react with the diaper's front section. For a diaper which only contains a urine-section, it is possible to attach the material to the back section of the diaper and still obtain adequate waist and leg elastics.

## Claims

1. An absorbent disposable article, such as a diaper or incontinence guard, comprising an inner, permeable enveloping layer (3), which on usage of the article lies closest to the user's body, an outer impermeable enveloping layer (4), as well as an absorbent body (2) placed between the enveloping layers, **characterized** by a material piece (5) of a thin, flexible material being attached to the outer enveloping layer (4) which material piece comprises a pattern of elastic threads (6,7,2,10) or elastic bands, being attached to the material piece (5) in a pretensioned state, and which material piece in the article's lengthwise direction extends beyond the back edge of the enveloping layers (3,4) encasing the absorbent body and in which the article's breadth-wise direction in its back section, has such an extension that when the article is put on by a user, the side edges of the material piece (5) are attachable to the corresponding side edge of the article's front section in order to give the applied article a pants-like configuration, whereby the pattern of elastic threads comprises threads (7 resp. 6) which form leg and waist elastics.

2. An article according to Claim 1, **characterized** by the material piece (5), as well as the waist and leg elastic forming threads (6 resp. 7) within the area of the absorbent body (2), also containing length-wise extending elastic threads (9, 10).

3. An article according to Claim 1, where the absorbent body and its enveloping layers have a rectangular form, **characterized** by the material piece having extended side-pieces in its front section, which together with the back section of the material piece form the waist section of the article when it is put on.

4. An article according to Claim 1 and 3, **characterized** by the material piece (5) comprising transverse elastic threads in its front section.

## Patentansprüche

1. Absorbierender Wegwerfartikel, beispielsweise eine Windel oder ein Inkontinenzschutz, mit einer inneren, flüssigkeitsdurchlässigen Deckschicht (3), die beim Gebrauch des Artikels auf den Körper der Benutzerperson zu weist, einer äußeren, flüssigkeitsundurchlässigen Deckschicht (4) sowie einem zwischen den Deckschichten angeordneten absorbierenden Körper (2),
**gekennzeichnet** durch ein Materialstück (5) aus dünnem, flexiblem Material, das an der äußeren Deckschicht (4) angebracht ist und ein Muster von elastischen Fäden (6, 7, 9, 10) oder elastischen Bändern aufweist, die am Materialstück (5) vorgespannt angebracht sind, wobei sich das Materialstück in Längsrichtung des Artikels über die hintere Kante der Deckschichten (3, 4) hinaus erstreckt und dabei den absorbierenden Körper einhüllt und in Breitenrichtung des Artikels in seinem hinteren Teil eine solche Ausdehnung hat, daß dann, wenn der Artikel von einer Benutzerperson getragen wird, die Seitenkanten des Materialstückes (5) an der entsprechenden Seitenkante des vorderen Abschnittes des Artikels befestigt werden können, um dem angezogenen Artikel eine hosenähnliche Form zu geben, wobei das Muster der elastischen Fäden Fäden enthält (7 bzw. 6), die elastische Bein- bzw. Bauchabschlüsse bilden.

2. Artikel nach Anspruch 1, dadurch gekennzeichnet, daß das Materialstück (5) innerhalb des Bereiches des absorbierenden Körpers (2) sowohl die elastischen Bauch- und Beinabschlüsse bildenden Fäden (6 bzw. 7) aufweist als auch elastische Fäden (9, 10), die sich in Längsrichtung erstrecken.

3. Artikel nach Anspruch 1, bei dem der absorbierende Körper und die ihn einhüllenden Deckschichten eine rechteckige Form haben, dadurch gekennzeichnet, daß das Materialstück (5) in seinem Vorderteil vorspringende Seitenteile hat, die zusammen mit dem rückwärtigen Teil des Materialstückes dann, wenn der Artikel angelegt ist, den Taillen- oder Bauchabschnitt bildet.

4. Artikel nach Anspruch 1 und 3, dadurch gekennzeichnet, daß das Materialstück (5) in seinem Vorderteil quer verlaufende, elastische Fäden aufweist.

## Revendications

1. Article absorbant jetable, comme une couche culotte ou une protection pour incontinent, qui comprend une couche d'enveloppe intérieure (3) perméable, qui, pendant l'utilisation de l'article, se trouve le plus près du corps de l'utilisateur, une couche d'enveloppe extérieure (4) imperméable, ainsi qu'un corps absorbant (2) placé entre les couches d'enveloppes, caractérisé par une pièce de matière (5) en matériau mince et souple, fixée à la couche d'enveloppe extérieure (4), pièce de matière qui comprend un réseau de fils élastiques (6, 7, 9, 10) ou de bandes élastiques qui sont fixés à la pièce de matière (5) à l'état pré-étiré, pièce de matière qui s'étend dans la direction de la longueur de l'article au-delà du bord arrière des couches d'enveloppes (3, 4) enfermant le corps absorbant, et qui a dans le sens de la largeur de l'article, au niveau de sa partie arrière, une dimension telle que lorsque l'article est mis en place par l'utilisateur, les bords latéraux de la pièce de matière (5) peuvent être fixés au bord latéral correspondant de la partie avant de l'article afin de donner à l'article mis en place une configuration de culotte, de sorte que le réseau de fils élastiques comprend des fils (7 resp. 6) qui consituent des élastiques de jambes et de taille.

2. Article selon la revendication 1, caractérisé par le fait que la pièce de matière (5), ainsi que les fils formant les élastiques de taille et de jambes (6 resp. 7), contient à l'intérieur de la région du corps absorbant (2) également des fils élastiques (9, 10) s'étendant dans le sens de la longueur.

3. Article selon la revendication 1, dans lequel le corps absorbant et ses couches d'enveloppe ont une forme rectangulaire, caractérisée par le fait que la pièce de matière a des parties latérales de prolongement dans sa partie avant, qui, avec la partie arrière de la pièce de matière, forment la partie taille de l'article lorsqu'il est mis en place.

4. Article selon les revendications 1 et 3, caractérisé par le fait que la pièce de matière (5) comprend des fils élastiques transversaux dans sa partie avant.
